(19) Europäisches Patentamt
European Patent Office
Office européen des brevets

(11) EP 2 332 467 A1

(12) **EUROPEAN PATENT APPLICATION**

(43) Date of publication:
**15.06.2011 Bulletin 2011/24**

(51) Int Cl.:
***A61B 8/00*** *(2006.01)*

(21) Application number: **10161016.0**

(22) Date of filing: **26.04.2010**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK SM TR**<br>Designated Extension States:<br>**AL BA ME RS**<br><br>(30) Priority: **14.12.2009 KR 20090123798**<br><br>(71) Applicant: **Medison Co., Ltd.**<br>**Kangwon-do 250-875 (KR)** | (72) Inventor: **Kim, Min Woo**<br>**151-050, Seoul (KR)**<br><br>(74) Representative: **Schmid, Wolfgang**<br>**Lorenz & Kollegen**<br>**Patentanwälte Partnerschaftsgesellschaft**<br>**Alte Ulmer Strasse 2**<br>**89522 Heidenheim (DE)** |

(54) **Ultrasonic 3-dimensional image reconstruction method and ultrasonic wave system thereof**

(57) Provided are an ultrasonic three-dimensional (3D) image reconstruction method and an ultrasonic wave system thereof. The ultrasonic 3D image reconstruction method may include forming, from data of ultrasound plane waves transmitted from a plurality of directions, a two-dimensional (2D) image having various directivities in a frequency space; forming 3D image data by applying a back-projection scheme to data of the 2D image; and reconstructing a 3D image by performing an inverse fast Fourier transformation (IFFT) on the 3D image data.

**FIG. 1**

START

INPUT PLANE WAVE DATA — S10

FORM 2D IMAGE — S11

FORM 3D IMAGE DATA — S13

RECONSTRUCT 3D IMAGE — S15

END

EP 2 332 467 A1

**Description**

CROSS-REFERENCE TO RELATED APPLICATION

This application claims the benefit of Korean Patent Application No. 10-2009-0123798, filed on December 14, 2009, in the Korean Intellectual Property Office, the disclosure of which is incorporated herein by reference.

BACKGROUND

1. Field of the Invention

The present invention relates to an ultrasonic three-dimensional (3D) image reconstruction method and an ultrasonic wave system using the same, and more particularly, to an ultrasonic 3D image reconstruction method and an ultrasonic wave system thereof, which may use a proj ection-slice theory.

2. Description of the Related Art

Along with an expanded use of an ultrasonic wave system, there are various demands for ultrasonic images provided by the ultrasonic wave system. In particular, since a lesion or a tissue of a patient should be more accurately estimated to perform medical operations such as diagnosis, biopsy, surgery, and the like, the ultrasonic wave system may need to acquire ultrasonic three-dimensional (3D) images.

To acquire the ultrasonic 3D images, a high Pulse Recurrence Frequency (PRF) scheme may be required. In particular, to display a rapidly moving object such as a heart of a human being in 3D images in real time, the high PRF scheme may be necessary. Thus, to realize the high PRF scheme, there have been suggested techniques using plane waves, which may obtain a plurality of scan-line data at once. However, since a focusing operation is not performed when using the plane waves, noise may be greatly generated due to a low Signal to Noise Ratio (SNR), and images having only a low resolution may be obtained.

Also, in a conventional 3D image reconstruction scheme, 3D images are reconstructed, using an interpolation technique, from acquired 2D images having various directivities, however, 3D images with only a relatively low resolution may be obtained due to increased noise in the 2D images and a limitation in a number of the 2D images.

SUMMARY

An aspect of the present invention provides an ultrasonic three-dimensional (3D) image reconstruction method and an ultrasonic wave system thereof, which may obtain ultrasonic 3D images having a high resolution.

According to an aspect of the present invention, there is provided an ultrasonic three-dimensional (3D) image reconstruction method, including: forming, from data of ultrasound plane waves transmitted from a plurality of directions, a two-dimensional (2D) image having various directivities in a frequency space; forming 3D image data by applying a back-projection scheme to data of the 2D image; and reconstructing a 3D image by performing an inverse fast Fourier transformation (IFFT) on the 3D image data.

According to another aspect of the present invention, there is provided an ultrasonic wave system, including: a 2D image formation unit to acquire, from ultrasound plane waves transmitted from a plurality of directions, a 2D image having various directivities in a frequency space; a 3D image reconstruction unit to acquire 3D image data by applying a back-projection scheme to data of the 2D image, and to reconstruct a 3D image by performing an inverse fast Fourier transformation (IFFT) on the 3D image data; and a display unit to display the 3D image.

EFFECT

According to embodiments of the present invention, it may be possible to obtain ultrasonic three-dimensional (3D) images having a high resolution from 2D images having various directivities, using a projection-slice theory.

BRIEF DESCRIPTION OF THE DRAWINGS

These and/or other aspects, features, and advantages of the invention will become apparent and more readily appreciated from the following description of exemplary embodiments, taken in conjunction with the accompanying drawings of which:

FIG. 1     is a flowchart illustrating an ultrasonic three-dimensional (3D) image reconstruction method according to an embodiment of the present invention;

FIG. 2     is a conceptual diagram illustrating a 2D image generation method using a Fast Fourier Transformation (FFT) according to an embodiment of the present invention;

FIG. 3     is a conceptual diagram illustrating a 2D image generation method using a lookup table according to an embodiment of the present invention;

FIG. 4     is a conceptual diagram illustrating a process of reconstructing 3D images from 2D images having various directivities in a frequency space according to an embodiment of the present invention; and

FIG. 5     is a block diagram illustrating a structure of an ultrasonic wave system that performs an ultrasonic 3D image reconstruction method according to an embodiment of the present invention.

DETAILED DESCRIPTION

Reference will now be made in detail to exemplary embodiments of the present invention, examples of which are illustrated in the accompanying drawings, wherein like reference numerals refer to the like elements throughout. Exemplary embodiments are described below to explain the present invention by referring to the figures.

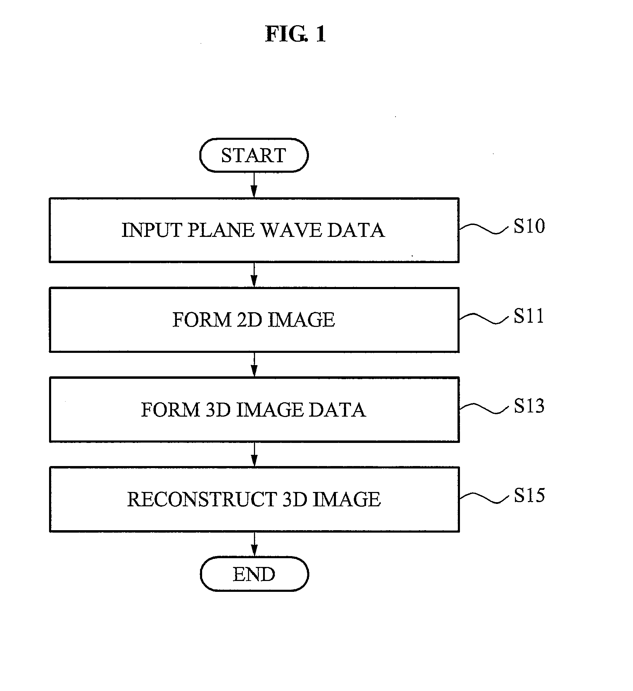

An ultrasonic three-dimensional (3D) image reconstruction method according to an embodiment of the present invention will be described in detail with reference to FIG. 1. FIG. 1 is a flowchart illustrating an ultrasonic 3D image reconstruction method according to an embodiment of the present invention.

Referring to FIG. 1, in operation S11, the ultrasonic 3D image reconstruction method may generate 2D images using plane wave data transmitted from a plurality of directions when the plane wave data is inputted in operation S10.

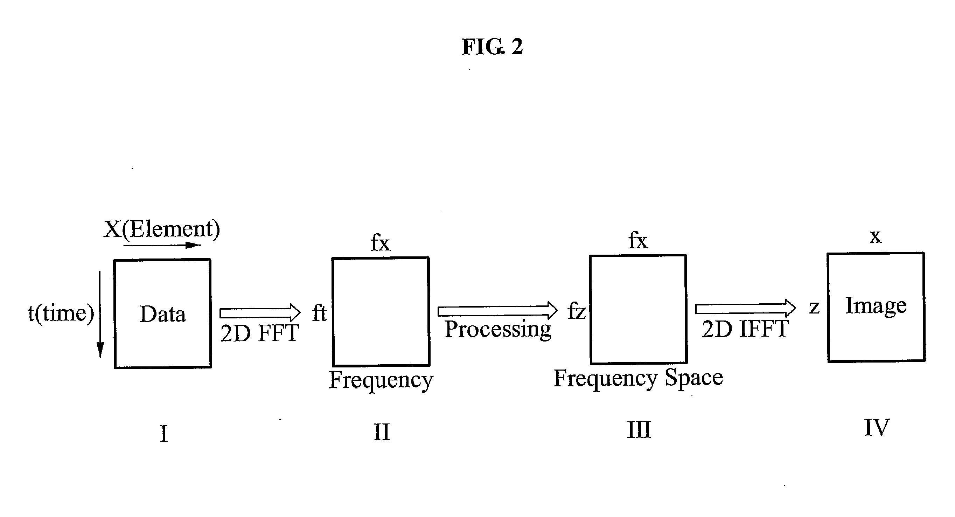

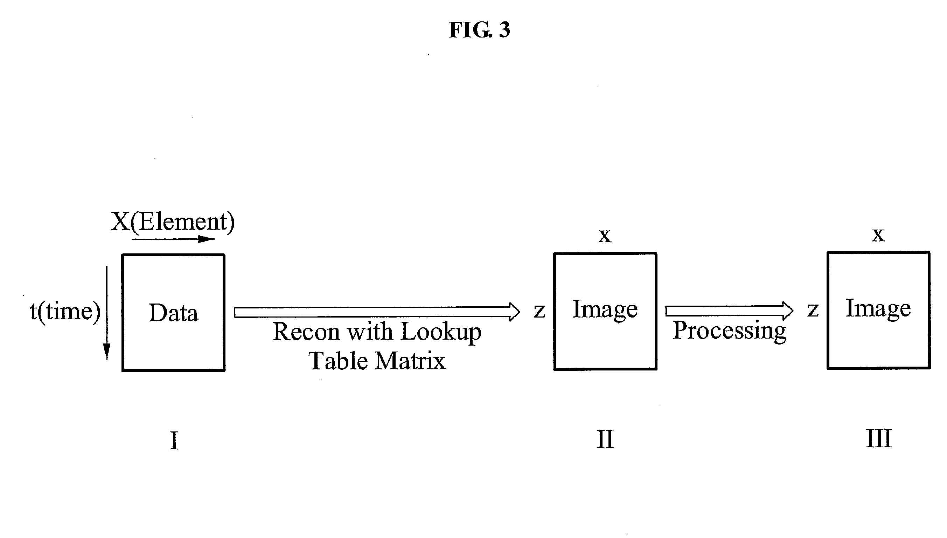

FIG. 2 is a conceptual diagram illustrating a 2D image generation method using a fast Fourier transformation (FFT) according to an embodiment of the present invention, and FIG. 3 is a conceptual diagram illustrating a 2D image generation method using a lookup table according to an embodiment of the present invention.

Referring to FIG. 2, plane wave data may be arranged on an X-axis (element) and a t-axis (time) as illustrated in stage I of FIG. 2, and then a 2D FFT may be performed to obtain values of 'fx' and 'ft' in a frequency domain, as illustrated in stage II of FIG. 2. Next, the obtained values of 'fx' and 'ft' may be subjected to a phase shift operation, an interpolation operation, and a remapping operation to thereby convert the values of 'fx' and 'ft' into values of 'fx' and 'fz' of an x-axis and an z-axis, as illustrated in stage III of FIG. 2. Finally, a 2D inverse fast Fourier transformation (IFFT) may be performed on the converted values( 'fx' and 'fz' ) to thereby generate 2D images, in stage IV of FIG. 2.

Referring to FIG. 3, the plane wave data of stage I of FIG. 2 may be arranged in the X-axis and the t-axis, as illustrated in stage I of FIG. 3, and then data where an Rx dynamic focusing operation is performed based on a lookup table matrix may be obtained as illustrated in stage II of FIG. 3. Next, the obtained data may be subjected to a phase shift operation, an interpolation operation, and a remapping operation to thereby generate 2D images, in stage III of FIG. 3.

Referring again to FIG. 1, in operation S13, the ultrasonic 3D image reconstruction method may generate 2D images, and then 3D image data may be formed using a back-projection scheme. Here, various methods of forming the 3D image data from the 2D images having various directivities may be provided. For example, assuming that 3D image data is 'x', a set of 2D image data having various directivities is 'y', and a projection matrix is 'A', the following Equation 1 may be obtained.

[Equation 1]

$$Y = Ax.$$

'x' may be obtained from 'y' using an inverse function of 'A', as illustrated in the following Equation 2.

[Equation 2]

$$\bar{x} = A^{-1}y = A^{T}Fy.$$

However, since a projection set is limited, a reconstruction result may be improved using a constraint. These series of processes may be performed using a projection-slice theory.

A compressed sensing scheme may be one of various signal processing schemes.

The compressed sensing scheme may be a technique for acquiring or reconstructing a signal utilizing the prior knowledge that is sparse or compressible. Based on the compressed sensing scheme, images having a higher resolution may be obtained from the limited projection set. For this, a value of minimizing noise using an L1 norm constraint may be obtained, as illustrated in the following Equation 3.

[Equation 3]

$$\bar{x} = \arg_{x} ||y - Ax||_{l_{z}} + \propto |x|_{l_{z}}.$$

Alternatively, using a de-noising technique, noise may be significantly reduced in the 2D image data.

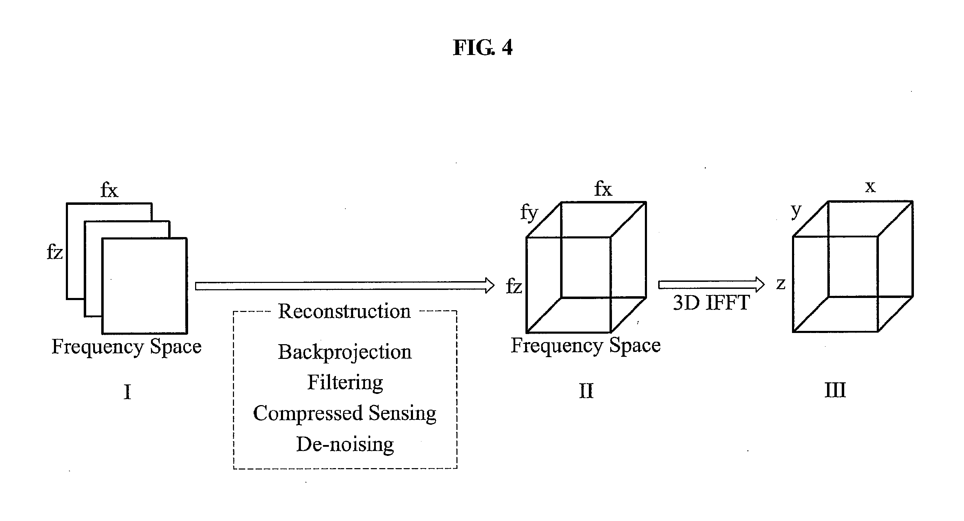

FIG. 4 is a conceptual diagram illustrating a process of reconstructing 3D images from 2D images having various directivities in a frequency space according to an embodiment of the present invention.

A plurality of 2D images having various directivities are illustrated in stage I of FIG. 4, and a process of forming 3D image data by applying a back projection, a filtering, a compressed sensing, a de-noising, and the like to each of the plurality of 2D image is illustrated in stage II of FIG. 4. Next, an ultrasonic 3D image is reconstructed by applying 3D-IFFT to the obtained 3D image, in stage III of FIG. 4.

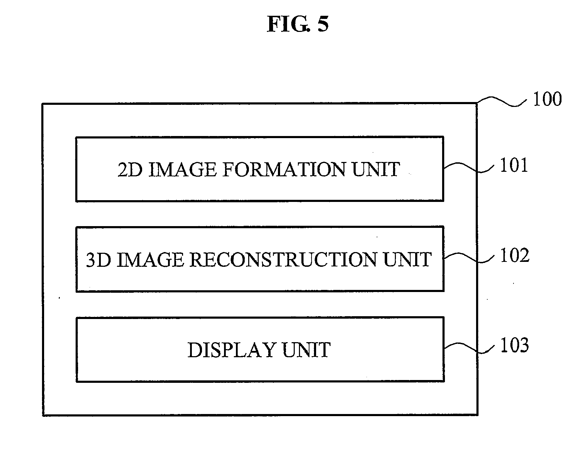

FIG. 5 is a block diagram illustrating a structure of an ultrasonic wave system 100 that performs an ultrasonic 3D image reconstruction method according to an embodiment of the present invention.

Referring to FIG. 5, the ultrasonic wave system 100 includes a 2D image formation unit 101 to acquire, from ultrasound plane waves transmitted from a plurality of directions, a 2D image having various directivities in a frequency space, a 3D image reconstruction unit 102 to acquire 3D image data by applying a back-projection scheme to data of the 2D image and to reconstruct a 3D image by performing an inverse fast Fourier transformation (IFFT) on the 3D image data, and a display unit 103 to display the 3D image.

The ultrasonic 3D image reconstruction method and the ultrasonic wave system thereof according to an embodiment of the present invention may provide 3D images having a high resolution, using plane waves.

The above-described embodiments of the present invention may be recorded in computer-readable media including program instructions to implement various operations embodied by a computer. The media may also include, alone or in combination with the program instructions, data files, data structures, and the like. The program instructions recorded on the media may be those specially designed and constructed for the purposes of the embodiments, or they may be of the kind well-known and available to those having skill in the computer software arts. Examples of computer-readable media include magnetic media such as hard disks, floppy disks, and magnetic tape; optical media such as CD ROM disks and DVD; magneto-optical media such as optical disks; and hardware devices that are specially configured to store and perform program instructions, such as read-only memory (ROM), RAM, flash memory, and the like. The media may also be a transmission medium such as optical or metallic lines, wave guides, etc. including a carrier wave transmitting signals specifying the program instructions, data structures, etc. Examples of program instructions include both machine code, such as produced by a compiler, and files containing higher level code that may be executed by the computer using an interpreter. The described hardware devices may be configured to act as one or more software modules in order to perform the operations of the above-described embodiments of the present invention, or vice versa.

Although a few exemplary embodiments of the present invention have been shown and described, the present invention is not limited to the described exemplary embodiments. Instead, it would be appreciated by those skilled in the art that changes may be made to these exemplary embodiments without departing from the principles and spirit of the invention, the scope of which is defined by the claims and their equivalents.

**Claims**

1.    An ultrasonic three-dimensional (3D) image reconstruction method, comprising:

   forming, from data of ultrasound plane waves transmitted from a plurality of directions, a two-dimensional (2D) image having various directivities in a frequency space;
   forming 3D image data by applying a back-projection scheme to data of the 2D image; and
   reconstructing a 3D image by performing an inverse fast Fourier transformation (IFFT) on the 3D image data.

2.    The ultrasonic 3D image reconstruction method of claim 1, wherein the forming of the 2D image forms the 2D image of the frequency space of the ultrasound plane waves using a fast Fourier transformation (FFT) or a lookup table.

3.    The ultrasonic 3D image reconstruction method of claim 1, wherein the forming of the 3D image data filters the data of the 2D image and then applies the back-projection scheme to the data of the 2D image.

4.    The ultrasonic 3D image reconstruction method of claim 1, wherein the forming of the 3D image data further applies a compressed sensing scheme and a de-noising scheme to the data of the 2D image to thereby acquire the 3D image data.

5.    An ultrasonic wave system, comprising:

   a 2D image formation unit to acquire, from ultrasound plane waves transmitted from a plurality of directions, a 2D image having various directivities in a frequency space;
   a 3D image reconstruction unit to acquire 3D image data by applying a back-projection scheme to data of the 2D image, and to reconstruct a 3D image by performing an inverse fast Fourier transformation (IFFT) on the 3D image data; and
   a display unit to display the 3D image.

6.    The ultrasonic system of claim 5, wherein the 2D image formation unit acquires the 2D image of the frequency space of the ultrasound plane waves using a fast Fourier transformation (FFT) or a lookup table.

7.    The ultrasonic system of claim 5, wherein the 3D image reconstruction unit filters the data of the 2D image and then applies the back-projection scheme to the data of the 2D image.

8.    The ultrasonic system of claim 5, wherein the 3D image reconstruction unit further applies a compressed sensing scheme and a de-noising scheme to the data of the 2D image to thereby acquire the 3D image data.

9.    At least one medium comprising computer readable instructions implementing the method of claim 1.

**FIG. 1**

START

INPUT PLANE WAVE DATA     ～ S10

FORM 2D IMAGE     ～ S11

FORM 3D IMAGE DATA     ～ S13

RECONSTRUCT 3D IMAGE     ～ S15

END

EP 2 332 467 A1

**FIG. 2**

X(Element)

t(time)

| Data |
|------|

2D FFT → ft

fx

| |
|--|

Frequency

Processing → fz

fx

| |
|--|

Frequency Space

2D IFFT → z

x

| Image |
|-------|

I

II

III

IV

FIG. 3

EP 2 332 467 A1

X(Element)

t(time)

| Data |

Recon with Lookup
Table Matrix

z | Image | x

Processing

z | Image | x

I

II

III

**FIG. 4**

EP 2 332 467 A1

FIG. 5

```
                                                          ⌇ 100
┌──────────────────────────────────────────────────┐
│  ┌────────────────────────────────────────────┐   │
│  │       2D IMAGE FORMATION UNIT              │   ⌇ 101
│  └────────────────────────────────────────────┘   │
│  ┌────────────────────────────────────────────┐   │
│  │   3D IMAGE RECONSTRUCTION UNIT             │   ⌇ 102
│  └────────────────────────────────────────────┘   │
│  ┌────────────────────────────────────────────┐   │
│  │          DISPLAY UNIT                       │   ⌇ 103
│  └────────────────────────────────────────────┘   │
└──────────────────────────────────────────────────┘
```

Europäisches
Patentamt

European
Patent Office

Office européen
des brevets

# EUROPEAN SEARCH REPORT

Application Number

EP 10 16 1016

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/123048 A1 (LEROUX JEAN-DANIEL [CA] ET AL) 14 May 2009 (2009-05-14) * figures 1,2,18 * * paragraphs [0063], [0066], [0071], [0073], [0121] - [0122], [0136], [0140] * | 1-9 | INV. A61B8/00 |
| Y | WO 2007/027703 A2 (UNIV TOLEDO [US]) 8 March 2007 (2007-03-08) * figure 1 * * paragraphs [0056] - [0059], [0063], [0066], [0067], [0094], [0154], [0269] * | 1-9 | |
| Y | US 2002/131546 A1 (OIKAWA SHIRO [JP]) 19 September 2002 (2002-09-19) * page 6 - page 8 * | 1-9 | |
| A | WO 02/48734 A2 (ESSEX CORP [US]; WOODFORD PAUL W [US]; FROEHLICH FRED F [US]) 20 June 2002 (2002-06-20) * figures 1,6 * * paragraphs [0055], [0069] - [0070], [0076], [0080] * | 1,3-5, 7-9 | TECHNICAL FIELDS SEARCHED (IPC) A61B G06T G01S |
| A | US 2006/102846 A1 (MANJESHWAR RAVINDRA M [US] ET AL MANJESHWAR RAVINDRA MOHAN [US] ET AL) 18 May 2006 (2006-05-18) * paragraphs [0023], [0026], [0030] - [0038] * * figures 1-3 * | 1,5,9 | |
| A | US 2009/141995 A1 (CHAKRABORTY AMIT [US] ET AL) 4 June 2009 (2009-06-04) * paragraphs [0037] - [0038] * | 4,8 | |

The present search report has been drawn up for all claims

| Place of search | Date of completion of the search | Examiner |
|---|---|---|
| The Hague | 30 September 2010 | Vanderperren, Yves |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 16 1016

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2010

| Patent document cited in search report | | Publication date | Patent family member(s) | | Publication date |
|---|---|---|---|---|---|
| US 2009123048 | A1 | 14-05-2009 | CA | 2631004 A1 | 09-11-2008 |
| WO 2007027703 | A2 | 08-03-2007 | US | 2009066727 A1 | 12-03-2009 |
| US 2002131546 | A1 | 19-09-2002 | JP<br>JP | 3518520 B2<br>2002263092 A | 12-04-2004<br>17-09-2002 |
| WO 0248734 | A2 | 20-06-2002 | AU | 3070302 A | 24-06-2002 |
| US 2006102846 | A1 | 18-05-2006 | NONE | | |
| US 2009141995 | A1 | 04-06-2009 | NONE | | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020090123798